# EUROPEAN PATENT SPECIFICATION

(11) **EP 1 095 205 B1**
(45) Date of publication and mention of the grant of the patent: **04.10.2006**
(21) Application number: 99932832.1
(22) Date of filing: 01.07.1999
(51) Int. Cl.: E21B 49/00, E21B 49/02, G01N 33/24, G01N 29/14

(54) **METHOD OF DETERMINING IN-SITU STRESSES IN AN EARTH FORMATION**
VERFAHREN ZUR BESTIMMUNG VON SPANNUNGEN AN ORT UND STELLE IN EINER IRDISCHEN FORMATION
PROCEDE PERMETTANT DE DETERMINER LA CONTRAINTE IN SITU D'UNE FORMATION GEOLOGIQUE

(30) Priority: 07.07.1998 EP 98202284
(43) Date of publication of application: 02.05.2001
(73) Proprietor: SHELL INTERNATIONALE RESEARCH MAATSCHAPPIJ B.V., 2596 HR Den Haag (NL)
(72) Inventor: KENTER, Cornelis, Jan, NL-2288 GD Rijswijk (NL); VAN MUNSTER, Johannes, Gerardus, NL-2288 GD Rijswijk (NL); PESTMAN, Barend, Jan, NL-2288 GD Rijswijk (NL)
(86) International application number: PCT/EP1999/004701
(87) International publication number: WO 2000/001927

(56) References cited:
- WO-A-97/36091
- DE-A- 2 927 529
- FR-A- 2 663 121
- US-A- 4 107 981
- US-A- 4 981 037
- US-A- 5 243 855
- US-A- 5 253 518
- US-A- 5 339 679
- B.J. PESTMAN: "An Acoustic Emission Study of Damage and Stress-Memory Effects in Sandstone" INTERNATIONAL JOURNAL OF ROCK MECHANICS AND MINING SCIENCES & GEOMECHANICS ABSTRACTS, vol. 33, no. 6, September 1996 (1996-09), pages 585-593, XP002116297 cited in the application

## Description

The present invention relates to a method of determining an in-situ stress of an earth formation, the formation being subjected to an in-situ stress state with a first, a second and a third principal stress. The three principal stresses are generally referred to as the first, the second and the third in-situ stress. In the technology of hydrocarbon production from an earth formation it is often required to know the magnitudes and directions of the in-situ stresses in the formation, or at least to have an indication thereof. Such knowledge is needed, for example, for the purpose of achieving wellbore stability, conducting hydraulic fracturing of the formation, geological modelling or preventing sand production. The direction of the in-situ stresses can be determined in several manners such as differential strain analysis, various acoustic techniques, or so-called minifrac tests. In this respect it is to be understood that one of the in-situ stresses is generally in vertical direction and its magnitude is determined from the weight of the overburden. Therefore, in general only the two horizontal in-situ stresses are subject of investigation with respect to direction and magnitude. It has been tried to determine the magnitudes of the horizontal in-situ stresses by measuring strains and using constitutive properties of the rock to determine the stresses. However, the constitutive properties of the rock are generally not accurately known.

B.J. pestman: "An acoustic emission study of damage development and stress-memory effects in sandstone", Int.Journal of Rock Mechanics and Mining Sciences & Geomechanics Abstracts; vol. 333, no. 6, Sept. 1996; p. 585-593, discloses a series of laboratory acoustic emisssion experiments on sandstone samples.

US-A-5,253,518 discloses methods for multi-stage tests of rock samples using isostatic pressure measuring the pore pressure of the sample.

US-A-4, 107, 981 discloses a method of estimating ground pressure in rock samples.

It is therefore an object of the invention to determine more accurately the magnitude of one or more of the in-situ stresses in the earth formation.

In accordance with the invention there is provided a method of determining an in-situ stress of an earth formation subjected to first, second and a third in-situ stresses, wherein a borehole has been drilled into the formation, the borehole containing a borehole fluid inducing a selected pressure to the borehole wall so that in a region of the formation the first in-situ stress is replaced by another stress depending on said selected pressure induced to the borehole wall, the method comprising the steps of:
- selecting a sample which has been removed from said region, the sample having first, second and third reference directions which coincide with the respective directions of the first, second and third in-situ stresses prior to removal of the sample from the formation; and
- conducting a plurality of tests on the sample whereby in each test the sample is subjected to selected stresses in the reference directions so as to determine a damage envelope of the sample and to determine from the damage envelope at least one of the second and third in-situ stresses, wherein the magnitude of the selected stress in the first reference direction is substantially equal to the magnitude of said another stress.

It is to be understood that in the context of the present invention the borehole wall includes both the cylindrical part of the borehole wall and the bottom of the borehole. An important aspect of the invention is that account is taken of the severest stress state to which the sample material has been subjected in order to determine the damage envelope. By 'severest stress state' is meant the stress state at which the sample material has undergone the largest amount of damage. For example, if the sample is taken from the borehole bottom, the severest stress state is considered to occur just before removing the sample from the formation whereby the magnitude of the vertical in-situ stress at the location of the sample is replaced by a vertical stress equal to the borehole fluid pressure at the borehole bottom plus the weight of the rock material between the borehole bottom and the location of the sample. If the rock material contains pore fluid, the pore fluid pressure is to be deduced from said vertical stress to find the effective vertical stress (which is the stress carried by the rock grains).

In such severest stress state the ratio of the difference between the horizontal in-situ stresses and the vertical stress, to the mean stress is at a maximum.

The damage envelope (also referred to as the damage surface) is formed by the points in three-dimensional stress space at which the onset of additional damage occurs upon further loading of the sample material. The damage surface can be accurately determined from acoustic emission by the sample material at the onset of additional damage. Such acoustic emission is generally referred to as the Kaiser effect as, for example, described in "An acoustic emission study of damage development and stress-memory effects in sandstone", B J Pestman et al, Int. J. Rock Mech. Min. Sci. & Geomech. Abstr., Vol. 33, No. 6, pp. 585-593, 1996.

The invention will be described hereinafter in more detail and by way of example with reference to the accompanying drawings in which
Fig. 1 schematically shows a cross-section of a borehole formed in an earth formation, as used in the method of the invention;
Fig. 1A schematically shows the in-situ stresses present in the earth formation;
Fig. 1B schematically shows a core sample taken from the earth formation; and
Fig. 2 schematically shows an in-situ stress diagram used in an embodiment of the method of the invention.

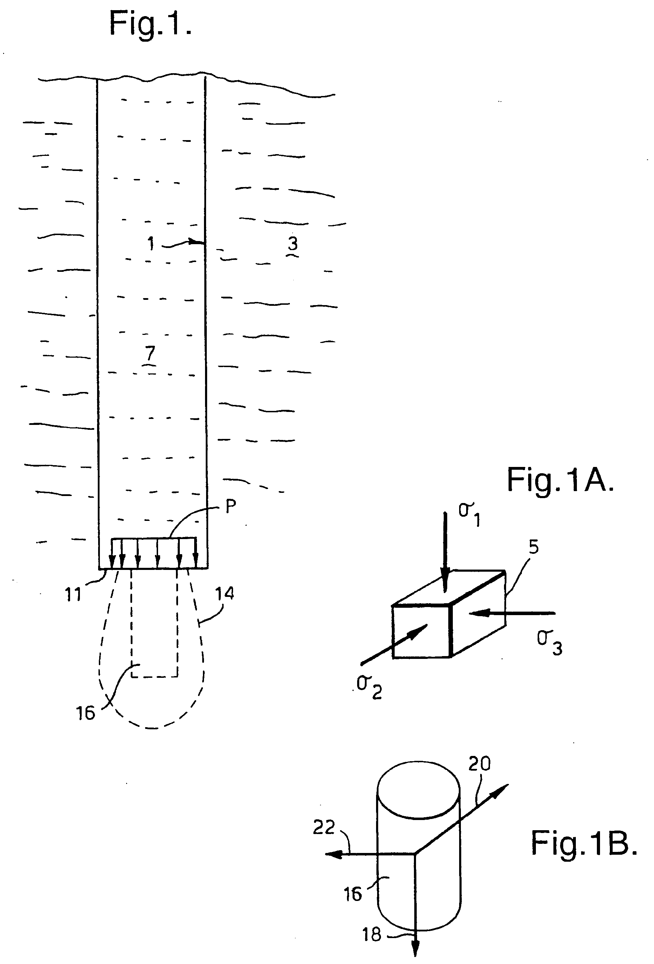

In the detailed description below it is assumed that the earth formation contains no pore fluid, hence the stresses referred to are effective stresses carried by the rock grains. Fig. 1 shows a borehole 1 formed in an earth formation 3. The undisturbed formation 3 is subjected to in-situ stresses in vertical and horizontal direction, i.e. a vertical compressive stress σ₁ and two horizontal compressive stresses σ₂, σ₃ as shown in Fig. 1A in relation to a cube-shaped element 5 of the formation 3. The borehole 1 is filled with a drilling fluid 7 of selected specific weight such that a vertical pressure P is exerted by the drilling fluid 7 on the borehole bottom 11. Below the borehole bottom 11 is a region 14 of the formation 3 in which the vertical in-situ stress σ₁ at a specific point is replaced by a stress σ₁' equal to the vertical pressure P from the drilling mud 7 plus the weight of the rock material between the borehole bottom 11 and the specific point. The horizontal in-situ stresses σ₂, σ₃ in region 14 are not (or only very little) affected by the presence of the borehole.

A coring tool (not shown) is lowered through the borehole 1 to take a cylindrical core sample 16 (Fig. 1B) from region 14 of the formation 3. In Fig. 1 the core sample 16 is indicated in dotted lines to show the location of the rock material of the core sample 16 prior to taking the sample 16 from the formation 3. The core sample 16 has a first reference direction 18, a second reference direction 20 and a third reference direction 22, which reference directions correspond to the respective in-situ stress directions prior to removal of the sample 16 from the formation 3. Thus, prior to removal of the sample 16 from the formation 3, reference direction 18 corresponds to vertical, reference direction 20 corresponds to the direction of in-situ stress σ₂ and reference direction 20 corresponds to the direction of in-situ stress σ₃. During and after removal of the core sample 16 from the formation 3 the compressive stresses acting in the reference directions are altered when the core sample 16 is stored in a container (not shown) containing a fluid at a moderate hydrostatic pressure.

In a next step a series of pressure tests are carried out on the core sample 16 whereby the sample is subjected to compressive stresses S₁, S₂, S₃ in respective reference directions 18, 20, 22. The purpose of the tests is to determine the amount of damage which the material of the core sample 16 has undergone prior to removal from the earth formation 3 and to estimate the horizontal in-situ stresses therefrom. The amount of damage can be represented by a damage envelope in three-dimensional stress space (S₁, S₂, S₃). Considering that the amount of damage of the sample material is determined by the severest stress state to which the sample material has been subjected (i.e. the stress state causing the largest amount of damage) it is an important aspect of the invention that it is taken into account that the severest stress state of the sample material occurred in the presence of the borehole 1 and prior to removing the sample 16 from the formation. Therefore in the severest stress state the principal stresses are σ₁' in reference direction 18, σ₂ in reference direction 20 and σ₃ in reference direction 22.

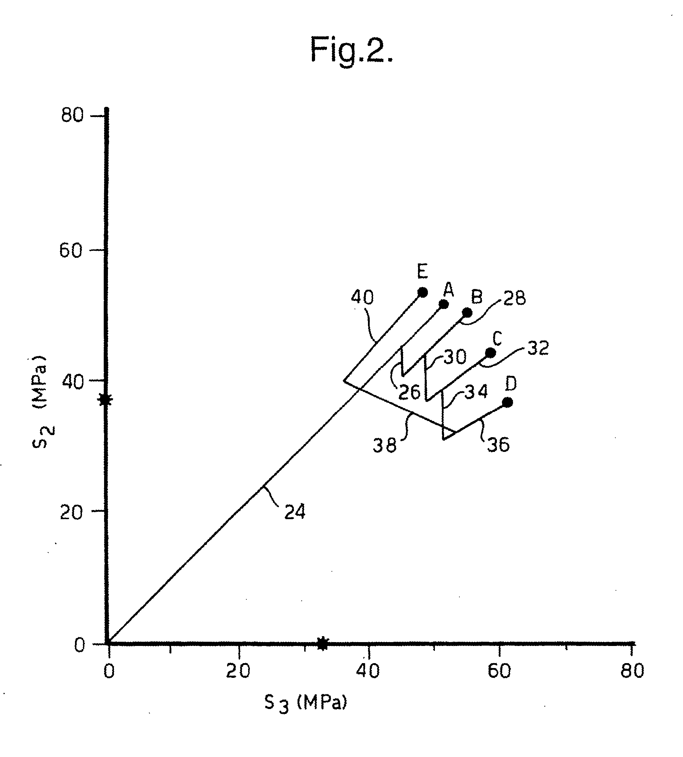

With reference to Fig. 2, the profile of the damage envelope for S₁ = σ₁' is then determined in a series of tests to estimate the magnitudes of horizontal in-situ stresses σ₂ and σ₃. During the tests the compressive stress S₁ is kept equal σ₁', while stresses S₂ and S₃ are varied until the onset of additional damage occurs. In the example diagram of Fig. 2 the sample 16 is loaded along stress path 24 to point A at which the onset of additional damage occurs. Such onset of additional damage is determined by measuring acoustic emission from the material, based on the Kaiser effect. Next the stresses S₂ and S₃ are changed along stress paths 26, 28 to point B, along stress paths 28, 30, 32 to point C, along stress paths 32, 34, 36 to point D, and along stress paths 36, 38, 40 to point E, whereby the points B, C, D, E are determined by the onset of additional damage in accordance with the Kaiser effect. The curve formed by points A, B, C, D, E make up the profile of the damage surface for S₁ = σ₁'· In conducting the tests, care is to be taken that the severest stress state of the sample material is not exceeded to a significant extent in order to ensure that the damage profile as determined from the tests accurately represents the severest stress state which occurred before the sample 16 was removed from the formation 3.

The damage profile in the S₁, S₂ diagram (for S₁ = σ₁') forms a set of points (S₁, S₂) of which each point could, in principle, represent the in-situ stress state (σ₁, σ₂, σ₃). A selection is made in a known manner to determine from these points the real in-situ stress state, for example by taking a vertex point in the profile as being representative for the real in-situ stresses state.

In case the rock material contains pore fluid, the total stress at a specific point in the formation is the sum of the effective stress (carried by the rock grains) and the pore fluid pressure. The above method then can be applied in a similar manner for the effective in-situ stresses σ₁ₑ, σ₂ₑ and σ₃ₑ. The vertical effective in-situ stress σ₁ₑ at a specific point is replaced by a stress σ₁ₑ' equal to the vertical pressure P from the drilling mud 7 plus the weight of the rock material between the borehole bottom 11 and the specific point minus the pore fluid pressure. The magnitudes of the horizontal effective in-situ stresses σ₂ₑ and σ₃ₑ are then determined in a similar manner as described above with reference to σ₂ and σ₃.

## Claims

1. A method of determining an in-situ stress of an earth formation (3) subjected to a vertical and two horizontal in-situ stresses (σ₁,σ₂,σ₃), wherein a borehole (1) has been drilled into the formation, the borehole (1) containing a borehole fluid exerting a pressure (P) to the borehole wall (11) so that in a region (14) of the formation the first in-situ stress (σ₁) is replaced by a reduced exerted stress depending on said pressure (P) induced to the borehole wall (11), the method comprising the steps of:
- selecting a sample (16) which has been removed from said region (14), the sample (16) having first, second and third reference directions (18,20,22) which coincide with the respective directions of the vertical and two horizontal in-situ stresses (σ₁,σ₂,σ₃) prior to removal of the sample (16) from the formation; and
- conducting a plurality of tests on the sample whereby the sample (16) is subjected to varying stresses (S₂,S₃) in the second and third reference directions so as to determine a damage envelope of the sample and to determine from the damage envelope at least one of the two horizontal in-situ stresses (σ₂,σ₃) and where in each test the magnitude of a selected stress (S₁) in the first reference direction (18) is substantially equal to the magnitude of said reduced stress.

2. The method of claim 1, wherein said region (14) is below the borehole bottom (11) and said reduced stress is defined by the weight of the fluid column in the borehole (1)and the weight of the part of the earth formation (3) between the borehole bottom (11) and the location where the sample (16) is removed from the formation (3).

3. The method of claim 1 or 2, wherein said sample (16) has been removed from the bottom region (14) of the borehole (1).

4. The method of any one of claims 1-3, wherein in each test a point (A,B,C,D,E) of the damage envelope is determined from acoustic emission from the sample (16).

## Patentansprüche

1. Verfahren zur Bestimmung einer in situ-Spannung einer Erformation (3), die einer vertikalen und zwei horizontalen in situ-Spannungen (σ₁, σ₂, σ₃) unterworfen ist, wobei ein Bohrloch (1) in die Formation gebohrt worden ist, das Bohrloch (1) ein Bohrlochfluid enthält, das einen Druck (P) auf die Bohrlochwand (11) aufbringt, so daß in einem Bereich (14) der Formation die erste in situ-Spannung (σ₁) durch eine reduzierte aufgebrachte Spannung ersetzt wird, abhängig von dem vorbestimmten Druck (P), der auf die Bohrlochwand (11) induziert wird, wobei das Verfahren die Schritte aufweist:
- Auswahl einer Probe (16), die aus dem Bereich (14) entfernt worden ist, wobei die Probe (16) erste, zweite und dritte Bezugsrichtungen (18, 20, 22) aufweist, die mit den entsprechenden Richtungen der vertikalen und zwei horizontalen in situ-Spannungen (σ₁, σ₂, σ₃) koinzidieren, bevor die Probe (16) aus der Formation entfernt worden ist; und
- Durchführen einer Vielzahl von Tests an der Probe, wobei die Probe (16) den ausgewählten Spannungen (S₂, S₃) in den zweiten und dritten Bezugsrichtungen unterworfen wird, um einen Schadensbereich der Probe und aus dem Schadensbereich zumindest eine der zwei horizontalen in situ-Spannungen (σ₂, σ₃) zu bestimmen, und wobei in jedem Test die Größe einer gewählten Spannung (S₁) in der ersten Bezugsrichtung (18) im wesentlichen gleich der Größe der reduzierten Spannung ist.

2. Verfahren nach Anspruch 1, bei welchem sich der Bereich (14) unterhalb des Bohrlochbodens (11) befindet, und die reduzierte Spannung durch das Gewicht der Fluidsäule in dem Bohrloch (1) und das Gewicht des Teiles der Erdformation (3) zwischen dem Bohrlochboden (11) und der Stelle bestimmt wird, an der die Probe (16) aus der Formation (3) entfernt wird.

3. Verfahren nach Anspruch 1 oder 2, bei welchem die Probe (16) aus dem Bodenbereich (14) des Bohrloches (1) entfernt worden ist.

4. Verfahren nach einem der Ansprüche 1-3, bei welchem in jedem Test ein Punkt (A, B, C, D, E) des Schadensbereiches aus der akustischen Emission der Probe (16) bestimmt wird.

## Revendications

1. Procédé permettant de déterminer une contrainte in situ d'une formation terrestre (3) soumise à une contrainte verticale et deux contraintes horizontales in situ (σ₁, σ₂, σ₃), dans lequel un trou de forage (1) a été foré dans la formation, le trou de forage (1) contenant un fluide de trou de forage exerçant une pression (P) sur la paroi (11) du trou de forage de sorte que, dans une région (14) de la formation, la première contrainte in situ (σ₁) est remplacée par une contrainte exercée réduite dépendant de ladite pression (P) induite sur la paroi (11) du trou de forage, le procédé comprenant les étapes consistant à :
- choisir un échantillon (16) qui a été extrait de ladite région (14), l'échantillon (16) ayant des première, deuxième et troisième directions de référence (18, 20, 22) qui coïncident avec les directions respectives de la contrainte verticale et des deux contraintes horizontales in situ (σ₁, σ₂, σ₃) avant l'extraction de l'échantillon de la formation, et
- effectuer une pluralité d'essais sur l'échantillon, l'échantillon (16) étant soumis à des contraintes variées (S₂, S₃) dans les deuxième et troisième directions de référence afin de déterminer une enveloppe endommagée de l'échantillon et afin de déterminer à partir de l'enveloppe endommagée au moins une des deux contraintes horizontales in situ (σ₂, σ₃) et où, dans chaque essai, la magnitude d'une contrainte choisie (S1) dans la première direction de référence (18) est sensiblement égale à la magnitude de ladite contrainte réduite.

2. Procédé de la revendication 1 dans lequel ladite région (14) se trouve en dessous du fond (11) du trou de forage et ladite contrainte réduite est définie par le poids de la colonne de fluide dans le trou de forage (1) et le poids de la partie de la formation terrestre (3) entre le fond (11) du trou de forage et l'emplacement où l'échantillon (16) est extrait de la formation (3).

3. Procédé de la revendication 1 ou 2 dans lequel ledit échantillon (16) a été extrait de la région (14) du fond du trou de forage (1).

4. Procédé de l'une quelconque des revendications 1 - 3 dans lequel, dans chaque essai, un point (A, B, C, D, E) de l'enveloppe endommagée est déterminé par émission acoustique de l'échantillon (16).
